# EUROPEAN PATENT SPECIFICATION

(11) **EP 0 231 914 B1**
(45) Date of publication and mention of the grant of the patent: **10.08.1994**
(21) Application number: 87101356.1
(22) Date of filing: 02.02.1987
(51) Int. Cl.: C07K 7/08, A61K 37/02, G01N 33/569

(54) **HTLV-III envelope peptides**
HTLV-III umhüllende Peptiden
Peptides d'enveloppe de HTLV-III

(30) Priority: 03.02.1986 US 824913
(43) Date of publication of application: 12.08.1987
(73) Proprietor: F. HOFFMANN-LA ROCHE AG, 4002 Basel (CH); THE GOVERNMENT OF THE UNITED STATES as represented by the Secretary of the Department of Commerce, Washington, D.C. (US)
(72) Inventor: Gallo, Robert Charles, Bethesda, MD 20834 (US); Heimer, Edgar Philip, Sparta, NJ 07871 (US); Reddy, Premkumar Eragam, Villanova, PA 19085 (US); Wong-Staal, Flossie, c/o Natl. Cancer Institute, Bethesda, MD 20205 (US)
(74) Representative: Lederer, Franz, Dr.

(56) References cited:
- EP-A- 0 201 716
- EP-A- 0 227 169
- WO-A-86/02383
- WO-A-88/00471
- CHEMICAL ABSTRACTS, vol. 106, 1987, Columbus, OH (US); T.C. CHARH et al., p. 376, no. 31045e#
- CHEMICAL ABSTRACTS, vol. 110, 1989, Columbus, OH (US); p. 639, no. 171673r#
- JOURNAL OF BIOLOGICAL CHEMISTRY, vol. 262, no. 12, 25 April 1987; R.C. KENNEDY et al., pp. 5769-5774#

## Description

This invention relates to synthetic peptides, designated HTLV-III env whose sequence represent a conserved region of the viral envelope protein of the etiologic agent for Acquired Immune Deficiency Syndrome (AIDS), derivatives of such peptides and the use of such peptides and/or derivatives in methods for detecting the presence of AIDS antibodies in human blood. Additionally, such peptides can be utilized in immunogenic compositions which can be used as vaccines or to elicit antibodies in host animals, which antibodies in turn can be employed to detect the HTLV-III virus in biological fluid specimens.

The results disclosed herein are based in part on the techniques and concepts of the field of immunology. For convenience, certain terms commonly used in the art are defined herein. The term "immunochemical reaction" is used to denote the specific interaction which occurs between an antigen and its corresponding antibody, regardless of the method of measurement. Such a reaction is characterized by a non-covalent binding of one or more antibody molecules to one or more antigen molecules. The immunochemical reaction may be detected by a large variety of immunoassays known in the art. The terms "immunogenic" or "antigenic" will be used here to describe the capacity of a given substance to stimulate the production of antibodies specifically immunoreactive to that substance when that substance is administered to a suitable test animal under conditions known to elicit antibody production. The term "protective antigen" refers to the ability of a given immunogen to confer resistance in a suitable host, against a given pathogen. The term "epitope", refers to a specific antibody binding site on an antigen. Macromolecular antigens such as proteins typically have several epitopes with distinctive antibody binding specificities. Different epitopes of the same antigen are distinguishable with the aid of monoclonal antibodies which, due to their high degree of specificity, are directed against singly epitopes. Two different monoclonal antibodies directed against different epitopes on the same antigen may each bind the antigen without interfering with the other, unless the epitopes are so close together that the binding of one sterically inhibits the binding of the other. The term "immunodominant region" denotes an area of the antigen molecule which is mainly responsible for its antigenicity.

From 1981 to date, there have been over thirteen thousand (13,000) people diagnosed as having acquired immune deficiency syndrome (AIDS). AIDS has been characterized by the onset of severe opportunistic infections secondary to an effect on the body's immune system [Gottlieb, M.S. et al., "Pneumocystis Carinii Pneumonia and Mucosal Candidiasis in previously healthy homosexual men: evidence of a new acquired cellular immuno-deficiency", N. Eng. J. Med. 305, 1425-1431 (1981)]. The disease has been found in male homosexuals, patients receiving blood products, intravenous drug addicts, and individuals originating from Haiti and Central Africa [Plot, P. et al, "Acquired immunodeficiency syndrome in a heterosexual population in Zaire", Lancet 11, 65-69 (1984)]. The causative agent was suspected to be of viral origin as the epidemiological pattern of AIDS was consistent with a transmissable disease. At least three (3) retroviruses have been isolated from cultured T-cells of several patients with AIDS, or from white blood cells of persons at risk for the disease. A novel human retrovirus called lymphadenopathy- associated virus (LAV) was discovered and its properties were consistent with its etiological role in AIDS. That virus was isolated from a patient with lymphadenopathy and hence the name [Montag- nier, L. et al., "A New Human T-lymphotropic retrovirus: Characterization and possible role in lymphadenopathy and acquired immune deficiency syndromes" in Human T-Cell Leukemia/Lymphoma Virus, R.C. Gallo, M. Essex and L. Gross, eds. (Cold spring Harbor, N.Y.: Cold Spring Harbor Laboratory 1984) pp. 363-370]. Other human retroviruses, specifically two subgroups of the human T-cell leukemia/lymphoma/lymphotropic virus, types I [Poiesz, B.J. et al. PNAS (USA) 77, 7415-7419 (1980)] and III [Popovic, M. et al., "Detection, isolation and continuous production of cytopathic retroviruses (HTLV-III) from patients with AIDS and pre-AIDS", Science 224, 497-500 (1984)] have also been isolated. Still another virus, the AIDS associated retrovirus (ARV), was proposed as the causative agent [Levy, J.A. et al., "Isolation of lymphocytopathic retroviruses from San Francisco patients with AIDS", Science 225, 840-842 (1984)]. Both the HTLV-III and ARV retroviruses display biological and sero-epidemiological properties similar to LAV [Levy et al., supra, Popovic et al., supra]. As seen from the above, at least three (3) retroviruses have been postulated as the etiologic agent of AIDS: LAV; ARV; and, HTLV subtypes I and III.

LAV, HTLV III and ARV-II genomes have been molecularly cloned [Schüpbach, J. et al., "Serological analysis of a subgroup of human T-lymphotropic retroviruses (HTLV III) associated with AIDS". Science 224, 503-505 (1984); Alizon, M. et al., "Molecular Cloning of lymphadenopathy - associated virus", Nature 312, 757-760 (1984)]. The complete nucleotide sequence of the proviral genome of LAV, ARV and HTLV-III has been determined [Ratner, L. et al., "Complete nucleotide sequence of the AIDS virus, HTLV III", Nature 313, 277-284 (1985); Sanchez-Pescador, R. et al., "Nucleotide sequence and expression of an AIDS-associated retrovirus (ARV-2), Science 227, 484-492 (1985); Wain-Hobson, S. et al., "Nucleotide sequence of the AIDS virus, LAV", Cell 40, 9-17 (1985)].

One reason for the difficulty in determining the etiologic agent of AIDS was due to the reactivity of various retroviral antigens with serum samples from AIDS patients. For example, serum samples from AIDS patients have been shown to react with antigens of HTLV I [Essex, M., et al., "Antibodies to Cell Membrane Antigens Associated with Human T-Cell Leukemia Virus in Patients with AIDS", Science 220, 859-862 (1983)]; and HTLV-lll [Sarngadharan, M.G. et al., "Antibodies Reactive With Human T-Lymphotropic RetroviruseS (HTLV-III) in the Serum of Patients With AIDS", Science 224, 506-508 (1984)]. Envelope gene products of HTLV demonstrated antigenicities cross-reactive with antibodies in sera from adult T-cell leukemia patients [Kiyokawa, T. et al., "Envelope proteins of human T-cell leukemia virus: Expression in Escherichia coli and its application to studies of env gene functions" PNAS (USA) 81, 6202-6206 (1984)]. Adult T-cell leukemias (ATL) differ from acquired immune deficiency syndrome (AIDS) in that HTLV-I causes T-cell malignancies that are characterized by uncontrolled growth of T-cells. In AIDS, instead of cell growth there is cell death. In fact this cytopathic characteristic of HTLV III was critical to determining ultimately the specific retroviral origin of the disease. Thus the etiologic agent of AIDS was isolated by use of immortalized human neoplastic T-cell lines (HT) infected with the cytopathic retrovirus characteristic of AIDS, isolated from AIDS afflicted patients. Seroepidemiological assays using this virus showed a complete correlation between AIDS and the presence of antibodies to HTLV III antigens [Sarngadharan et al., supra; Schüpbach et al., supra]. In addition, nearly 85% of patients with lymphadenopathy syndrome and a significant proportion of asymptomatic homosexual men in AIDS endemic areas were also found to carry circulating antibodies to HTLV III. Taken together, all these data indicate HTLV III to be the etiologic agent for AIDS.

peptides of the instant invention encompass a highly conserved epitope sequence designated HTLV-III env (500-511) which permit their use to screen for, diagnose and/or prevent by vaccination the AIDS virus.

### DESCRIPTION OF THE INVENTION

The peptides of the present invention can be represented by the following formula: 
W-X-Lys-Ala-Lys-Arg-Arg-Val-Val-Gln-Arg-Glu-Lys-
Arg-Y-Z (I)
 wherein W is H-, Cys- or Tyr-; X is a bond or a sub-sequence of one or more amino acids starting from the carboxyl terminus of HLTV-lll env (460-499); Y is a bond or a sub-sequence of one or more amino acids starting from the amino terminus of HTLV-III env (512-550); and Z is -OH, -NH₂ or -Cys-NH₂, provided however that one of X or Y is a bond.

Another patent application (WO 86/06414) has been published after the filing date of the present application designating the same European countries except Spain. This application discloses also peptides derived from HTLV-III proteins. One of these peptides [Peptide (Vlllb) (50)] overlaps with the peptides of the present invention and has been disclaimed in the corresponding set of claims.

The derivatives of the peptide of formula I having cysteine at either the amino or the carboxy terminus are utilized to provide improved coupling of the peptide to immunogenic carrier materials when the peptide is to be utilized as an immunogen to elicit antibody formation. The derivatives of formula I, wherein W is tyrosine, provide a preferred substrate for radio-iodination thereby allowing such compounds to be utilized as radiolabelled ligands in a radioimmunoassay for HTLV-III antibody in test fluids.

A preferred aspect of the compounds of the present invention is represented by compounds of formula I wherein X is a sub-sequence of one or more amino acids starting from the carboxyl terminus of HLTV-III env (460-499) and Y is a bond. A preferred embodiment of this aspect of the invention is obtained when said sub-sequence consists of HLTV-III env (487-499). Thus particularly preferred species of this embodiment consist of HLTV-III env (487-511) Cys-NH₂ and HLTV-III env Tyr (487-511) Cys-NH₂.

In another preferred aspect of the compounds of the present invention compounds of formula I wherein both X and Y are bonds are provided. Preferred embodiments of this aspect of the invention include HTLV-III env (500-511) Cys-NH₂, HTLV-III env Cys-(500-511) NH₂ and HTLV-III env Tyr (500-511) NH₂.

Compounds of the present invention of formula I can be conveniently prepared by utilizing conventional peptide synthesis methods well-known in the art. Such procedures include solution phase synthesis and solid phase synthesis applying the methodology developed by R.B. Merrifield [J. Am. Chem. Soc. 85, 2149-2154 (1963)]. In the solid phase synthetis method, which is the preferred methodology for synthesizing the compounds of the present invention of formula I, a polymeric solid-phase support to which the amino acids used in the synthesis are covalently anchored in sequence starting from the carboxyl terminus is used. Automated solid-phase peptide synthesizers suitable for carrying out such methodology are commercially available and synthesis is carried out according to the instructions of the manufacturer. In the event an amide group is desired at the carboxyl terminus for compounds of formula I, then the resin employed is a benzylhydrylamine resin which is an article of commerce. Cleavage of the peptide chain from such resin results in the formation of an amide group as desired at the carboxyl terminus.

Upon completion of the automated peptide synthesis, the desired peptides are removed from the resin utilizing methods well-known in the art, e.g. anhydrous liquid hydrogen fluoride (HF). This treatment also cleaves off any side chain protecting groups. The peptides can then be purified in conventional manner, such as, for example, by utilizing high performance liquid chromatography preferably with a reverse phase C₁₈ type column.

### Detailed Description of the Invention

In accordance with the present invention, the search for the env protein of the etiologic agent for Acquired Immune Deficiency Syndrome (AIDS) has lead to the isolation and sequencing and expressing of the proviral gene of the AIDS virus. It has now been discovered, that the postulated etiologic agents of AIDS, lymphadenophathy-associated virus (LAV), AIDS-associated - retrovirus (ARV) and human T-cell leukemia/lympha/lymphotropic virus, HTLV-III, are in fact variants of the same virus. For purposes of this invention and claims, the virus causing AIDS will be referred to herein as AIDS virus. AIDS virus will be understood to include the variants which have been postulated as the causative agent of AIDS, namely, LAV, ARV and HTLV-III. The env protein of the AIDS virus (env AIDS) is a 97,200 dalton protein with 32 potential N-glycosylation sites. Nucleotide sequence analysis of the AIDS env. gene of the putative etiologic agents of AIDS demonstrates that all the viruses are variants of the same virus. There has been found to be approximately 1 to 20% divergents of variation from the sequence from the env. gene of HTLV-III and the sequences of the env. genes of the other viruses LAV and ARV-II.

The integrated proviral genome of HTLV-III was cloned from the genomic DNA of H9 cells infected with HTLV-III [see Shaw et al. , Science 226, 1165-1171 (1984)]. The comparative nucleotide sequences of LAV, ARV-II and HTLV-III have been determined by Ratner, Nature 313, 277-284 (1985); Sanchez-Pescador et al., Science 227, 484-492 (1985); and Wain-Hobson et al., Cell 40, 9-17 (1985), respectively.

One of the conserved regions in these various viral env gene sequences is the region corresponding to amino acids 460-550 which contains the important epitopic site around 500-511. It is thus believed that such region provides a preferred antigenic site for use in detection of the presence of AIDS viruses or antibodies directed thereto present in the sera of human subjects. Of particular importance are the processing sites located within amino acids 500-511 which undergo proteolytic cleavage in the viral envelope. The resulting two peptides have portions of the general conserved regions embodied in amino acids 460-550 at their carboxyl and amino termini, respectively, thus allowing antibody specific to this epitopic area raised from the peptides of the invention to recognize both processed proteins. Moreover, peptides constructed with such sequences would have superior chances to provide vaccines which could immunize populations against all three AIDS viral entities.

In one diagnostic embodiment of the present invention, the peptides of formula I can be employed in a sandwich type enzyme immunoassay to directly detect the presence of antibodies to AIDS virus in serum samples. Such assay can be carried out quite simply by coating microtiter plates with a dilute solution of a peptide of formula I, preferably when W is H and Z is -NH₂ or Cys-NH₂. The peptide coated microtiter plates can then be incubated with the test sample for sufficient time to allow any antibody present to complex with the peptide. After the incubation period has been completed, the serum sample is removed, the plates are washed and then treated with anti-human IgG coupled to an enzyme such as horseradish peroxidase. Such reagents are articles of commerce. If any antibody to AIDS virus is present in the serum sample, it will be bound by the peptide and will be complexed to the anti-human IgG bearing the enzyme label. After removal of the reagent, substrate for the enzyme is added and allowed to incubate in the microtiter plate. If antibodies were present in the sample, a color reaction will be observed in the microtiter plate.

In an alternative diagnostic embodiment, compounds of formula I wherein W is tyrosine are radioiodinated in a conventional manner such as, for example, by use of chloramine-T utilizing ¹²⁵1 as the radioactive ligand. Antibodies specific for AIDS virus can be obtained by utilizing compounds of formula I where either W or Z is cysteine as haptens in the preparation of an immunogenic composition. Such immunogenic composition is prepared by bonding the aforesaid haptens through the indicated cysteine moiety to a conventional immunogenic carrier material. As used herein, the term "immunogenic carrier material" is meant to include those materials which have the property of independently eliciting an immunogenic response in a host animal and which can be covalently coupled to the above described haptens preferably through said cysteine moiety. Suitable carrier materials include, for example, proteins: natural or synthetic polymeric compounds, such as, polypeptides, e.g., polylysine or copolymers of amino acids, polysaccharides; and the like. Particularly preferred carrier materials are proteins and polypeptides, especially proteins.

The identity of the protein material utilized in the preparation of an immunogen useful in the instant invention is not critical. Examples of suitable proteins useful in the practice of this invention include mammalian serum proteins such as, for example, human gamma globulin, human serum albumin, bovine serum albumin, methylated bovine serum albumin, rabbit serum albumin and bovine gamma globulin. A particularly preferred protein for this purpose is keyhole limpet hemocyanin.

The covalent coupling of the peptide hapten to the immunogenic carrier material can be carried out in the manner well-known in the art. Reagents suitable for this purpose include N-succinimide esters, carbodiimides, EEDQ(N-ethoxycarbonyl-2- ethoxy-1,2-dihydroquinoline) and the like. A particular preferred reagent for use in coupling the peptide hapten to the keyhole limpet hemocyanin of the preferred embodiment is m-maleimido- benzoyl-N-succinimide ester.

The aforesaid immunogen may be utilized to induce formation of antibodies specific to AIDS virus in host animals by injecting the immunogen in such a host preferably using an adjuvant known in the art such as complete or incomplete Freund's adjuvant. Improved titers can be obtained by repeated injections over a period of time. Suitable host animals for this purpose include mammals such as, rabbits, horses, goats, guinea pigs, rats, cows, sheep, etc. The resulting antiserum will contain antibodies which will selectively complex with AIDS virus, e.g. HTLV-III preparations containing the env (460-550) epitope. The antiserum can be affinity purified in a manner known per se by passing such anti-serum over an affinity column prepared from a peptide of formula I, preferably where W is Cys, coupled to a solid support matrix such as Sepharose.

In a further aspect of this invention it is possible to produce monoclonal antibodies specific to the aforesaid 460-550 epitope of the env gene of HTLV-III. The production of monoclonal antibodies can be carried out according to the general procedure described by Kohler and Milstein [Nature 256, 495-597 (1975)]. The resulting hybrid cells produced by this method have the property of secreting an antibody of predefined specificity. This specificity is that of the antibody produced by lymphocytes involved in the fusion. The hybrid cells may be cloned and grown in stable culture to produce in the culture supernatant samples of antibody to a specific determinant or epitope.

The general method of production of hybrid cell lines of the type described above comprises the steps of immunizing an animal (usually a rat or mouse but not necessarily one of those) with the described immunogen, i.e. a peptide of formula I wherein W or Z is cysteine covalently bonded to an immunogenic carrier material. After allowing time for the immune system to generate lymphocytes secreting the antibody to the immunogen, the animal is sacrificed and a suspension of the spleen is prepared. Fusion between these cells and myeloma cells is achieved by bringing them into contact in the presence of a fusion promoter (e.g. polyethylene glycol). A small percentage of the cells fuse to produce hybrid myeloma cells. The immunization results in a plurality of different lymphocytes each secreting antibody to a different antigenic determinant, and these characteristics are transferred genetically to the hybrid cells. It is possible, by careful screening, to isolate from a culture of hybrid cells, a cell having the desired specificity to the 460-550 epitope of the env region of the HTLV-III virus. Such cells may be cloned and cultured by methods now well-known in the art so as to provide a continuous source of the desired monoclonal antibody.

Any conventional immuno assay method now known in the art may be employed using the antibodies of the invention. For example, a suitable radioimmunoassay method utilizing the above described reagents can be carried out by mixing a sample containing the HTLV-III virus with a known amount of the labelled tyrosine analog of formula I and the HTLV-III (460-550) env protein specific monoclonal or polyclonal antibody, measuring the degree of binding of the labelled analog and determining the amount of AIDS virus present in said sample by comparing said degree of binding to a standard curve obtained by mixing known amounts of AIDS virus with fixed amounts of said labelled analog and said antibody and determining the degree of binding for each known amount of virus or viral protein.

Another diagnostic embodiment of the invention provides a radioimmune assay for detecting the presence of AIDS antibodies in sera. In such assay sera samples are serially diluted in buffer and mixed with a radio iodinated analog of a peptide of formula I where W is Tyr. After incubation, anti-human IgG is added and the mixture incubated again. After centrifugation, the radioactivity bound to the precipitated antibody complex is measured. The presence of AIDS antibodies is detected by count values in excess of those observed as background for normal patient sera.

In a further diagnostic aspect of the invention, an immunometric assay procedure may be run utilizing the antibodies of the present invention produced above. In a representative embodiment of such assay method duplicate samples are run. The procedure employs 100 /1.1 of a suspension of antibody immobilized on Agarose particles mixed with 100 µl of serum and 100 µl of soluble ¹²⁵1- labelled antibody. This mixture is allowed to incubate for a time period ranging from 1/4 hour to 24 hours. Following the incubation, the Agarose particles are washed by addition of buffer and then centrifuged. After removal of the washing liquid by aspiration, the resulting pellet of Agarose particles is then counted for bound ¹²⁵1-labeled antibody. The counts obtained for each of the complexes can then be compared to controls.

The methods for the determination of AIDS virus or of antibodies against AIDS virus as described above can be conducted in suitable test kits comprising in a container a peptid of the present invention or antibodies against AIDS virus elicited by an immunogen composition of the present invention.

In a further aspect of the present invention, the peptides of formula I particularly those having W or Z as cysteine can be incorporated into vaccines capable of inducing protective immunity against the AIDS virus. Known techniques may be employed for enhancing the antigenicity of such peptide vaccine preparations, for example, by coupling such peptide through the cysteine moiety to a toxoid such as diptheria toxoid or tetanus toxoid. It is also possible to couple such peptides to inorganic carrier materials which enhance immunogenicity by presenting such peptides to the host subject in the manner approximating the normal antigen on the viral surface. Such inorganic carrier materials include aluminum hydroxide. It is within the skill of the art to use such vaccine composition in combination with adjuvants or other enhancers of immune response, such as, immune interferon, interleukin-2, thymosin alpha 1 and the like. In addition, the vaccine composition may comprise other antigens to provide immunity against other diseases in addition to AIDS, such as, for example, hepatitis core or surface antigens. It is also within the skill of the art to produce a vaccine composition incorporating the peptides of the present invention into liposomes as a means of maintaining the peptides in a desirable orientation for inducing immunity in a host.

Routes of administration, antigen dose, number and frequency of injections are all matters of optimization within the scope of ordinary skill in the art, particularly in view of the fact that there is experience in the art in providing protective immunity by injection of other related antigens to provide immunity in other viral infections. It is anticipated that the principal value of providing immunity to AIDS infection will be for those individuals who have had no previous exposure to AIDS, e.g., individuals who are in the high risk population, such as, homosexuals, drug addicts, Hatians and Central Americans, and individuals who may be receiving blood transfusions. It is also anticipated that temporary immunity for infants may be provided by immunization of mothers during pregnancy.

Because anti-HTLV-III antibodies are found in more than 90% of the AIDS patients, it was of interest to see if the synthetically produced peptides of the present invention are immunoreactive with these antibodies. For this analysis purified peptide of formula I wherein W is H and Z is Cys-NH₂ was transferred to a nitrocellulose filter by using Western blotting technique. Strips of the filter containing transferred peptide were reacted with 1,000-fold diluted human sera and the antigen-antibody complex formed as detected by incubation of the strips with ¹²⁵1-labelled Staphylococus aureus protein A followed by autoradiography. Bands corresponding to the peptide were consistently observed when the serum used was from patients with AIDS syndrome. The negative controls used were normal human sera and serum from a patient with HTLV-I infection. No reaction was observed with sera from healthy individuals or from HTLV-I infected individuals. Patient sera was derived from all parts of the United States including California and all AIDS patients sera tested, so far, were found to be positive. These results indicate that the epitopic region corresponding to positions 460-550 of the envelope protein are recognized by antibodies found in AIDS patients.

In further experiments it was found that polyclonal antibodies elicited from the same peptides coupled to keyhole limpet hemocyanin elicited in rabbits will immunoblot with recombinant HTLV-III env protein.

The above results indicate that peptides of formula I, as described above, will be suitable for use in the diagnostic assays and vaccine compositions described.

The present invention is further illustrated by the following examples. In such examples the abbreviation Tos means tosylate 2-CIZ means 2-chlorocarbobenzoxy, OBzl means O-benzyl and Dmb means dimethylbenzyl. Boc means benzox- ycarbonyl, Dcb is 2,6-dichlorobenzyl.

### Example 1

### Preparation of Boc-Cys(Dmb)-Benzhyldrylamine- resin (1)

Benzhydrylamine-resin (BHA) (20 g, 0.25 mmol/g) was coupled with Boc-Cys(Dmb)-OH (13.24g, 40.7mmol) in CH₂CI₂ (250 ml) with dicyclohexyl carbodiimide (DCC) (8g) for 4 hours. The resultant Boc-Cys(Dmb)-BHA- resin was washed with CH₂CI₂ (3 x 300ml), N,N-dimethylformamide (DMF) (2 x 300ml), MeOH (3 x 300ml), CH₂CI₂ (3 x 300ml) and dried. An aliquot was hydrolyzed (1 ml of 6M propionic HCI at 130°C for 2 hours). Amino acid analysis showed a substitution of 0.52 mmol of Cys per gram of resin. The remaining amino groups were acetylated with AC₂0-pyridine. Yield: 28.75g.

### Example 2

### Preparation of HTLV-III env (500-511)-Cys-NH₂ (2)

Boc-Cys(Dmb)-BHA-resin (2.0g, 1.0mmol) from Example 1 was charged into the reaction vessel of the Vega 250 Peptide Synthesizer and solid phase synthesis was performed by the symmetric anhydride procedure (Merrifield, R.B., supra) for a total of 12 cycles with the following N"-Boc amino acids:
Arg(Tos), Lys(2-CIZ), Glu(OBzl), Arg(Tos), Gln, Val, Val, Arg(Tos), Arg(Tos), Lys(2-CIZ), Ala and Lys(2-CIZ) to give 3.9g of protected peptide resin. The protected peptide resin (1.5g) was treated with anhydrous liquid HF containing 10% dithioethane of 0°C for 1 hour. The HF was evaporated at 0°C (high-vac, CaO trap) and the crude peptide and resin mixture triturated with EtOAC, extracted with TFA. evaporated and the residue triturated with ether and dried. Yield: 700mg. A part of the crude material (350mg) was dissolved in 10ml of H₂0 containing 0.5% trifluoroacetic acid (TFA), filtered (0.45µ type HA Millipore@ filter) and loaded onto a Nucleosil C 18 (5µ) column (1 x 50 cm).

The column was eluted (2ml/min.) with a solvent system consisting of (A) H₂O (containing 0.1 % TFA) and (B) CH₃CN (containing 0.1% TFA) in a linear gradient mode of 5-25% (B) in 180 minutes with a flow rate of 2ml/min. Fractions were collected (2 minutes/fraction) and aliquots analyzed by the analytical hplc system. The product which emerged in fractions 54-55 were combined, evaporated and lyophilized to give pure HTLV-III env (500-511)-Cys-NH₂. Yield: 8.5mg. The product was shown to be homogeneous by analytical hplc and gave the expected amino acid composition (hy-
^{*} Low value due to difficult Val-Val cleavage. drolysis in 6N HCI, 1% thioglycolic acid (TGA); 110°C; 72 hours): Glu, 2.04; Ala, 1.00; Val, 1.10^{*}; Lys, 2.94; Arg. 3.98. Sequence analysis confirmed the amino acid sequence to be HTLV III env (500-511 )-Cys-NH₂.

### Example 3

### Conjugation of HTLV-III env (500-511)-Cys-NH₂ with Keyhole Limpet Hemocyanin (KLH) (3)

Keyhole limpet hemocyanin, (Calbiochem, 4mg in 50% glycerol) was added to a solution of m-maleimidobenzoyl-N-succinimide ester (0.7mg) in DMF (0.1 ml) at 25°C and stirred (magnetically) for 30 minutes. The reaction mixture was charged onto a Sephadex@ G-25 column (1.2 x 25 cm) which was previously equilibrated with 0.05M NaH₂ PO₄ -(pH 6.0). Fractions (1ml/min.) were collected and the first major peak (280 nm detection) was pooled [MB-KLH complex]. The HTLV-III env (500-511)-Cys-NH₂ peptide (5.0mg, 3.0 mmol) in 1 ml of 0.05M NaH₂PO₄ (pH 6.0) was added to the solution containing the MB-KLH complex. The pH was adjusted to 7-7.5 by addition of 1.0N NaOH (µl amounts) and stirring proceeded at 25°C for 3 hours. The reaction mixture was dialyzed (MW cutoff 3500) against Dulbecco's phosphate buffered saline, with repeated changes at 4° C over a 60 hour period. The dialyzed solution (8.9ml) was used directly for antibody generation.

### Example 4

### Preparation of Boc-Arq(Tos)-Benzhydrylamine-resin (4)

Benzhydrylamine-resin (40.44g, 0.5 mmol/g, 20.22 mmol) was coupled with Boc Arg (Tos)-OH-(34.6g, 80.7 mmol) in 25% DMF-CH₂CI₂ (250 ml) with dicyclohexylcarbodiimide (DCC) (16.78g, 81.4 mmol) for 2h followed by the addition of 1% diisopropylethylamine and reacted for 0.5 h longer. The resultant Boc-Arg (Tos)-BHA-resin was washed with CH₂CI₂ (3 x 250 ml), MeOH(3 x 250 ml), CH₂CI₂ (3 x 250 ml) and dried. The couplings and washing procedures were repeated and an aliquot was hydrolyzed (1 ml of 6M propionic acid/HCI at 130°C for 2 h). Amino acid analysis showed a substitution of 0.35 mmol of Arg per gram of resin. The remaining amino groups were acetylated with AC₂0-Pyridine. Yield 48.88g (Boc-Arg(Tos)-BHA-resin, 4).

### Example 5

### Preparation of HTLV-lll env Cys-(500-511)-BHA-resin (5)

Boc-Arg(Tos)-BHA-resin (4, 2.0g, 0.7 mmol) was charged into the reaction vessel of the Vega 250 Peptide Synthesizer and 12 cycles of solid phase synthesis performed as in Example 2. At the end of the synthesis the BOC-group of the N-terminal amino acid residue was removed (TFA) and dried to give 2.8g of protected HTLV-III env Cys-(500-511 )-BHA-resin, 5.

### Example 6

### Preparation of HTLV-lll env Cys-(500-511)-NH₂ (6)

A 1.5g portion of protected peptide resin 5, was treated with anhydrous liquid HF as in Example 2. Yield: 741 mg. A portion of this material (350 mg) was loaded onto dual Nucleosil C-18 (5µ) columns (1 x 50 cm) and the columns were eluted (2 ml/min) with a solvent system consisting of (A) H₂0 (containing 0.1% TFA) and (B) CH₃CN (containing 0.1% TFA) in a linear gradient mode of 5-25% (B) in 180 min. Fractions were collected (2 min/fraction) and the aliquots analyzed by the analytical hplc system. The product emerged in fraction 48 which was evaporated and lyophilized. Yield: 46.1 mg (13%). The product was shown to be homogeneous by analytical hplc and gave the expected amino acid composition (hydrolysis in 6NHCI-1 % TGA; 110 ° C; 72h): Glu, 2.00; Ala, 1.04; Val, 1.36^{*}; Lys, 2.97; Arg 3.98.

### Example 7

### Preparation of HTLV-lll env Tyr (500-511)-NH₂ (7)

Boc-Arg(Tos)-BHA-resin, (2.0g, 0.7 mmol) was subjected to 12 cycles of peptide synthesis as in Example 2 to give 2.5g of protected HTLV-III env Tyr (500-511 )-BHA-resin. A portion of this material (1.5g) was cleaved with HF (as in Example 2) yielding 781 mg of crude peptide which was purified by hplc as in Example 6. The product emerged in fractions 66-68 which were pooled, evaporated and lyophilized. Yield: 42-5 mg (12%). The product was shown to be homogeneous by analytical hplc and gave the expected amino acid composition (hydrolysis in 6NHCI-1 %TGA; 110 ° C; 24h): Glu, 2.10; Ala, 1.04; Val, *0.90; Tyr, 0.86; Lys,
^{*} Low value due to the expected incomplete Val-Val cleavage.
*Low value due to the expected incomplete Val-Val cleavage. 2.89; Arg, 4.10.

### Example 8

### Preparation of of HTLV-lll env (487-511 )cys-BHA-resin (8)

A 2.2g portion of protected HTLV-III env (500-511 )-Cys-BHA-resin from Example 2, was subjected to 13 cycles of solid phase peptide synthesis, treated with TFA and dried to yield 1.8g of side chain protected HTLV-lll env (487-511 )-BHA-resin, 8.

### Example 9

### Preparation of HTLV-lll env (487-511)-Cys-NH₂ (9)

A 1.0 g portion of 8 was treated with anhydrous liquid HF, as in Example 2. Yield: 435 mg. The crude product was loaded into dual (in series) Synchropals columns (RP-8, 1 x 25 cm). The columns were eluted (2ml min) with a solvent system consisting of (A) H₂0 (containing 0.025% TFA) and (B) CH₃CN (containing 0.025%TFA) in a linear gradient mode of 5-25% (B) in 120 min. Fractions were collected (2 min/fraction) and aliquots analyzed by the analytical hplc system. The product emerged in fractions 77-82 which were combined, evaporated and lyophilized. Yield 31.1 mg (12%). The product was shown to be homogeneous by analytical hplc and sequence analysis confirmed the amino acid sequence to be HTLV env (487-511 )-Cys-NH₂.

### Example 10

### Conjugation of HTLV-lll env (487-511)-Cys NH₂ with Keyhole Limpet Hemocyanin (10)

Conjugation of 5 mg of 9 to keyhole limpet hemocyanin-MB complex (4 mg) was carried out exactly as in Example 3. The final dialyzed solution (8 ml) was used directly for antibody generation and for detection of HTLV-lll env protein antibodies.

### Example 11

### Synthesis of HTLV-lll env Tyr-(487-511)-Cys-NH₂ -(11)

An 0.5g portion of side chain protected HTLV-III env (487-511)-Cys-BHA-resin, 8, was subjected to 1 cycle of solid phase peptide synthesis using Boc-Tyr(Dcb), treated with HF as in example 2 and purified by hplc (as in Example 9). Yield: 15 mg. The product, 11, was homogeneous by analytical hplc.

### Example 12

### ELISA (Enzyme-Linked Immunosorbance Assay)

Human sera and rabbit sera were screened for anti-peptide antibodies by an ELISA assay. Peptides of Examples 2, 6 and 9 were dissolved in bicarbonate-carbonate saline solution (0.05 M bicarbonate-carbonate, pH 9.6; 0.15 NaCI) to give a concentration of 1 µ.g/ml. 100 microliters of this solution were pipeted into each microtiter well and the plates dried overnight at 37° C. Each well was then washed three times with PBS/tween@ buffer (0.02 M sodium phosphate buffer, pH 6.5; 0.15 M NaCI; 0.05% tween-20). The empty microtiter wells were then filled with blocking buffer (10% calf serum; 1% BSA; 0.3% gelatin; 0.5 M NaCI; 20 mM sodium phosphate, pH 7.5; 0.02% sodium azide) to block unreacted sites. After incubation for 1 hr at 37° C, the plates were washed with PBS/Tween@ buffer and 100 microliters of various dilutions (100 to 10,000) of test sera were added to the wells. Following incubation at room temperature for 2 hr, the plates were washed with PBS/Tween@ buffer. Color reactions were then performed by incubating each well with 100 µl of a 1 to 2000 diluted goat anti-human antibody conjugated with horse radish peroxidase. Incubations were carried out for 1 hr, the plates washed with PBS/Tween@ buffer and 100 µl of substrate solution added. The substrate solution was prepared by dissolving o-phenylene diamine dihydrochloride in 0.1 M citrate buffer (pH 4.5) at a concentration of 400 µg/ml and 4 µl of hydrogen perioxide added to 10 ml of this solution. Color was allowed to develop for approximately 30 min at 37° C. The reaction was stopped by the addition of 50 µl of 2.5 M H₂S0₄ and 50 mM Na₂S₂0s per well and absorption read at 488 nm on a microplate reader.

### Example 13

### Radioimmune Assay

For the radioimmune assay, the amino terminus tyrosine peptides of Examples 7 and 11 were labelled with ¹²⁵1 using chloramine T as a catalyst (Greenwood et al., Biochemical Journal, vol. 89, pp. 114-123, 1963). For this procedure, approximately 100 ng of the peptide was dissolved in 50 µl of 0.01 M Tris-HCI buffer. pH 7.5, containing 10 mM NaCI. 1 mC of radioactive iodine (¹²⁵1) was added to the solution followed by 10 µl of Chloramine T solution (2.5 mg/ml). The reaction was carried out for 1 min and the reaction stopped by the addition of 25 µl of sodium meta bisulfite (2.5 mg/ml) solution. The free ¹²⁵1 was then removed by column chromatography on a Biogel@ P-10 column.

For the radioimmune assay, the AIDS patient sera are diluted serially in Buffer I (10 mM tris-HCI buffer, pH 7.8 containing 2% BSA, 0.6 mg/ml of NaCI, 1% EDTA and 0.5% Triton@ X100). 100 µl of each dilution was pipeted into a test tube and mixed with 10,000 cpm of ¹²⁵l-labelled peptide. The mixture was incubated for 3 hr at 37 ° C. 50 µl of goat anti-human antibody was then added to the reaction mixture followed by 1 ml of rinse buffer (0.01 M Tris-HCI buffer pH 7.8, 0.1% Triton@ X100, 0.1 M NaCl and 1 mM EDTA). The mixture was then incubated for 1 hr at 37°C and for 2 hr at 4 °C. The tubes were then centrifuged at 2500 rpm and the supernatant discarded. The radioactivity bound to the human antibody was measured in a gamma counter. Counts in excess of the levels provided by normal sera indicated the presence of AIDS antibody in the test sera.

### Example 14

### Preparation of Rabbit Antisera Specific for the HTLV-lll Peptides

For the production of rabbit antibodies, the peptide conjugated to KLH was mixed with complete Freund's adjuvant or incomplete Freund's adjuvant in 1:1 proportion. On day 1, 200 µg of the conjugated peptide in complete Freund's adjuvant were injected into the rabbits intradermally on the back at multiple sites. On day 28, 200 µg of the peptide-KLH conjugate in incomplete Freund's adjuvant was injected intradermally on the back at multiple sites. On day 42, a 40 ml test bleed was taken and the antibody titer determined by ELISA assay. The rabbits were boosted again on day 56, with peptide-KLH conjugate in incomplete Freund's adjuvant followed by exsaugination at day 70.

### Example 15

### Affinity Purification of Anti-Peptide Antiserum to Peptide env 500-511

1. 14 ml of rabbit serum were chromatographed on a column of protein A-Sepharose (0.7 cm x 20 cm) followed by elution with 0.1 M citrate, pH 3.5. The acid eluate was neutralized and dialyzed against PBS. The yield of IgG was 88 mg as determined by absorbance at 280 nm. This is a typical value. The purity of the IgG preparation was determined by immunoelectrophoresis. Essentially all the IgG was removed from the serum by the protein A column. This IgG was obtained in pure form in the acid eluate.
2. An affinity column was constructed using HTLV-lll env Cys-(500-511)-NH₂. 8 mg of peptide was reacted with 0.4ml of epoxy-Sepharose@ followed by blocking with ethanolamine. The final coupling yield was 4.2 mg peptide/0.4 ml Sepharose as determined by amino acid analysis. This is about 35% of the theoretical maximum for this resin.
3. 70 mg of the IgG (80% of the sample) was chromatographed on the peptide-Sepharose@ column followed by elution with 0.2 M glycine, pH 2.5 and neutralization of the acid eluate. The serum was run over the column a second time and the combined acid eluates were dialyzed against PBS. The total yield of affinity purified IgG was 1.8 mg (2.5%). This is a reasonably good yield of specific anti-peptide antibody.
4. The extent to which the affinity purification procedure enriched the anti-peptide activity was assessed by ELISA. The criteria which were applied were the retention of anti-peptide reactivity together with the depletion of anti-KLH reactivity. The results showed that the reactivity of the affinity purified IgG toward synthetic peptide compares favorably with that of total IgG and serum. The results also show that the reactivity of the affinity purified IgG toward KLH has dropped to undetectable levels. The anti-KLH activity is all found in the peptide-column breakthrough.
5. To test whether the affinity-purified rabbit serum reacts with the HTLV-III envelope gene product, Western blot analysis was performed. For this analysis, bacterial cell lysates containing recombinant HTLV-III envelope protein were subjected to electrophoresis on an acrylamide gel and the proteins transferred to a nitrocellulose filter using Western blotting technique. Strips of the filter were reacted with 1000-fold diluted rabbit serum (affinity purified) and the antigen-antibody complex formed was detected by incubation of the strips with ¹²⁵1-labelled Staphylocous aureus protein A followed by autoradiography. Bands were observed corresponding to the envelop protein. The negative controls used were normal bacterial lysates which did not contain HTLV-lll expression plasmids. No bands were detected in the latter instance.

## Claims (Claims for the following Contracting State(s) : BE, CH, DE, FR, GB, IT, LI, NL, SE)

1. A peptide of the formula 
W-X-Lys-Ala-Lys-Arg-Arg-Val-Val-Gln-Arg-Glu-
Lys-Arg-Y-Z        (I)
 wherein W is H-, Cys-, or Tyr-; X is a bond or a sub-sequence of one or more amino acids starting from the carboxyl terminus of HTLV-III env (460-499); Y is a bond or a sub-sequence of one or more amino acids starting from the amino terminus of HTLV-lll env (512-550); and Z is -OH, -NH₂ or -Cys-NH₂, provided, however, that one of X or Y is a bond and X is other than Pro-Thr.

2. The peptide of claim 1 wherein both X and Y are bonds.

3. The peptide of claim 2, wherein W is H- and Z is -Cys-NH₂.

4. The peptide of claim 2 wherein W is Cys and Z is -NH₂.

5. The peptide of claim 2, wherein W is Tyr-.

6. A compound of claim 5 labelled with ¹²⁵1.

7. The peptide of claim 1 wherein X is a sub-sequence of one or more amino acids starting from the carboxyl terminus of HTLV-III env (460-499) and Y is a bond.

8. The peptide of claim 7 wherein X is HTLV-III env (487-499).

9. The peptide of claim 8 which is HTLV-III env (487-511 )-Cys-NH₂.

10. The peptide of claim 8 which is HLTV-lll env Tyr-(487-51 )-Cys-NH₂.

11. An immunogenic composition comprising a peptide of claim 1, wherein one of W or Z is Cys covalently bonded to an immunogenic carrier material.

12. The composition of claim 11, wherein W is Hand Z is -Cys-NH₂.

13. The composition of claim 11, wherein said immunogenic carrier material is keyhole limpet hemocyanin.

14. A peptide of claim 1 as constituent of a vaccine.

15. A peptide of claim 1 as constituent of an immunogenic composition.

16. A process for the preparation of a peptide of claim 1, characterized in that conventional peptide synthesis methods are used.

17. A process according to claim 16, characterized in that solid phase synthesis methods are used.

18. A process for the preparation of an immunogenic composition of claim 11, characterized in that a peptide of claim 1, wherein one of W or Z is Cys, is covalently bonded to an immunogenic carrier material.

19. A process according to claim 18, characterized in that a peptide of claim 1 wherein W is Hand Z is -Cys-NH₂ is used.

20. A process according to claim 18 or 19, characterized in that as an immunogenic carrier material Keyhole limpet hemocyanin is used.

21. A method of testing human blood for the presence of antibodies to the viral etiological agent of AIDS which comprises contacting a peptide of claim 1 which is bound to a solid support with a test sample of human blood whereby any antibodies to the viral etiological agent of AIDS present in said test sample will bind to said peptide, removing said test sample, washing said solid support to remove unbound antibody and probing said solid support with a reagent capable of selectively detecting human antibodies.

22. The method of claim 21, wherein said solid support is a plastic microtiter plate and said reagent capable of detecting human antibodies is anti-human IgG label led with horseradish peroxidase which is reacted with a substrate which provides a color change end point in the presence of said peroxidase.

23. The method of claim 21, wherein said peptide of claim 1 is HTLV-III env (487-511)-Cys-NH₂.

24. The method of claim 21, wherein said peptide of claim 1 is HTLV-lll env Cys-(500-511)-NH₂.

25. A process for the preparation of a vaccine comprising mixing a peptide of claim 1 with a physiologically acceptable carrier.

26. A process according to claim 25, wherein said peptide is coupled to a toxin, which is utilized to confer immunity in humans.

27. A non-therapeutic process for the preparation of antibodies against AIDS virus comprising injecting a host with a sufficient amount of an immunogen of claims 11 to 13 and recovering said antibodies from the serum of said host.

28. A vaccine against AIDS comprising a peptide of claim 1 in a physiologically acceptable medium.

29. The vaccine of claim 28, wherein said peptide is coupled to a toxin utilized to confer immunity in humans.

30. An antibody elicited by immunizing a host with an immunogen of claims 11 to 13, which selectively binds to a compound of claim 1, HTLV-III env protein or AIDS virus.

31. The antibody of claim 30, which is a polyclonal antibody.

32. The antibody of claim 30, which is a monoclonal antibody specific to the epitope HTLV-III env (460-550).

33. The use of a peptide of claim 1 for the preparation of a protective immunization vaccine.

34. The use of a peptide of claim 1 for testing human blood for the presence of antibodies to the AIDS virus.

35. The use of an immunogen composition of claims 11 to 13 for the preparation of antibodies which selectively bind to a compound of claim 1, HTLV-III env protein or AIDS virus.

36. A peptide as claimed in claim 1 prepared by a process as claimed in claim 16 or 17.

37. An immunogen composition as claimed in any one of claims 11 to 13 prepared by a process as claimed in any one of claims 18 to 20.

38. A test kit for testing human blood for the presence of antibodies to the AIDS virus comprising in a container a peptide of claim 1.

## Claims (Claims for the following Contracting State(s) : AT)

1. A process for the preparation of a peptide of the formula 
W-X-Lys-Ala-Lys-Arg-Arg-Val-Val-Gln-Arg-Glu-
Lys-Arg-Y-Z        (I)
 wherein W is H-, Cys-, or Tyr-; X is a bond or a sub-sequence of one or more amino acids starting from the carboxyl terminus of HTLV-III env (460-499); Y is a bond or a sub-sequence of one or more amino acids starting from the amino terminus of HTLV-lll env (512-550); and Z is -OH, -NH₂ or -Cys-NH₂, provided. however, that one of X or Y is a bond and that X is other than Pro-Thr, by conventional peptide synthesis methods.

2. A process according to claim 1, characterized in that solid phase synthesis methods are used.

3. A process according to claim 2, characterized in that protecting groups and/or the solid phase resins introduced during solid phase synthesis of a peptide of claim 1 are cleaved by conventional means.

4. A process according to claim 3, characterized in that the cleavage is cone by treatment with anhydrous liquid hydrogen fluoride.

5. A process according to any one of claims 1 to 4, characterized in that a peptide as defined in claim 1, wherein both X and Y are bonds, W is H- and Z is -Cys-NH₂, is prepared.

6. A process according to any one of claims 1 to 4, characterized in that a peptide as defined in claim 1, wherein both X and Y are bonds, W is Cys and Z is -NH₂, is prepared.

7. A process according to any one of claims 1 to 4, characterized in that a peptide as defined in claim 1, wherein both X and Y are bonds and W is Tyr-, is prepared.

8. A process according to any one of claims 1 to 4, characterized in that a peptide as defined in claim 1, wherein X is a sub-sequence of one or more amino acids starting from the carboxyl terminus of HTLV-III env (460-499) and Y is a bond, is prepared.

9. A process according to any one of claims 1 to 4, characterized in that a peptide as defined in claim 1, wherein X is HTLV-III env (487-499) and Y is a bond, is prepared.

10. A process according to any one of claims 1 to 4, characterized in that a HTLV-III env (487-511)-Cys-NH₂ peptide is prepared.

11. A process according to any one of claims 1 to 4, characterized in that a HTLV-III env Tyr-(487-511)-Cys-NH₂ peptide is prepared.

12. A process for the preparation of an immunogen composition characterized in that a peptide as defined in claim 1, wherein W or Z is Cys, is covalently bonded to an immunogenic carrier material.

13. A process according to claim 12, characterized in that a peptide as defined in claim 1 having W as H- and Z as -Cys-NH₂ is used.

14. A process according to claim 12 or 13, characterized in that said peptides are bounded to said immunogenic carrier material via a conventional bifunctional linking group.

15. A process according to claims 12 to 14, characterized in that as an inmunogenic carrier material Keyhole limpet hemocyanin is used.

16. A method of testing human blood for the presence of antibodies to the viral etiological agent of AIDS which comprises contacting a peptide as defined in claim 1 which is bound to a solid support with a test sample of human blood whereby any antibodies to the viral etiological agent of AIDS present in said test sample will bind to said peptide, removing said test sample, washing said solid support to remove unbound antibody and probing said solid support with a reagent capable of selectively detecting human antibodies.

17. The method of claim 16, wherein said solid support is a plastic microtiter plate and said reagent capable of detecting human antibodies is anti-human IgG labelled with horseradish peroxidase which is reacted with a substrate which provides a color change end point in the presence of said peroxidase.

18. The method of claim 16, wherein said peptide as defined in claim 1 is HTLV-III env (487-511 )-Cys-NH₂.

19. The method of claim 16, wherein said peptide as defined in claim 1 is HTLV-III env Cys-(500-511 )-NH₂.

20. A process for the preparation of a vaccine comprising mixing a peptide as defined in claim 1 with a physiologically acceptable carrier.

21. A process according to claim 20, wherein said peptide is coupled to a toxin, which is utilized to confer immunity in humans.

22. A non-therapeutic process for the preparation of antibodies against AIDS virus comprising injecting a host with a sufficient amount of an immunogen as defined in claims 12 to 15 and recovering said antibodies from the serum of said host.

23. The use of a peptide as defined in claims 1 and 5 to 11 for the preparation of a protective immunization vaccine.

24. The use of a peptide as defined in claims 1 and 5 to 11 for testing human blood for the presence of antibodies to the AIDS virus.

25. The use of an immunogen composition as defined in claims 12 to 15 for the preparation of antibodies which selectively bind to a compound as defined in claims 1 and 5 to 11, HTLV-III env protein or AIDS virus.

26. A test kit for testing human blood or the presence of antibodies to the AIDS virus comprising in a container a peptide as defined in claims 1 and 5 to 11.

## Claims (Claims for the following Contracting State(s) : ES)

1. A process for the preparation of a peptide of the formula 
W-X-Lys-Ala-Lys-Arg-Arg-Val-Val-Gln-Arg-Glu-
Lys-Arg-Y-Z (I)
 wherein W is H-, Cys-, or Tyr-; X is a bond or a sub-sequence of one or more amino acids starting from the carboxyl terminus of HTLV-III env (460-499); Y is a bond or a sub-sequence of one or more amino acids starting from the amino terminus of HTLV-lll env (512-550); and Z is -OH, -NH₂ or -Cys-NH₂, provided, however, that one of X or Y is a bond, by conventional peptide synthesis methods.

2. A process according to claim 1, characterized in that solid phase synthesis methods are used.

3. A process according to claim 2, characterized in that protecting groups and/or the solid phase resins introduced during solid phase synthesis of a peptide of claim 1 are cleaved by conventional means.

4. A process according to claim 3, characterized in that the cleavage is done by treatment with anhydrous liquid hydrogen fluoride.

5. A process according to any one of claims 1 to 4, characterized in that a peptide as defined in claim 1, wherein both X and Y are bonds, W is H- and Z is -Cys-NH₂, is prepared.

6. A process according to any one of claims 1 to 4, characterized in that a peptide as defined in claim 1, wherein both X and Y are bonds, W is Cys and Z is -NH₂, is prepared.

7. A process according to any one of claims 1 to 4, characterized in that a peptide as defined in claim 1, wherein both X and Y are bonds and W is Tyr-, is prepared.

8. A process according to any one of claims 1 to 4, characterized in that a peptide as defined in claim 1, wherein X is a sub-sequence of one or more amino acids starting from the carboxyl terminus of HTLV-III env (460-499) and Y is a bond, is prepared.

9. A process according to any one of claims 1 to 4, characterized in that a peptide as defined in claim 1, wherein X is HTLV-III env (487-499) and Y is a bond, is prepared.

10. A process according to any one of claims 1 to 4, characterized in that a HTLV-III env (487-511)-Cys-NH₂ peptide is prepared.

11. A process according to any one of claims 1 to 4, characterized in that a HTLV-III env Tyr-(487-511)-Cys-NH₂ peptide is prepared.

12. A process for the preparation of an immunogen composition characterized in that a peptide as defined in claim 1, wherein W or Z is Cys, is covalently bonded to an immunogenic carrier material.

13. A process according to claim 12, characterized in that a peptide as defined in claim 1 having W as H- and Z as -Cys-NH₂ is used.

14. A process according to claim 12 or 13, characterized in that said peptides are bounded to said immunogenic carrier material via a conventional bifunctional linking group.

15. A process according to claims 12 to 14, characterized in that as an immunogenic carrier material Keyhole limpet hemocyanin is used.

16. A method of testing human blood for the presence of antibodies to the viral etiological agent of AIDS which comprises contacting a peptide as defined in claim 1 which is bound to a solid support with a test sample of human blood whereby any antibodies to the viral etiological agent of AIDS present in said test sample will bind to said peptide, removing said test sample, washing said solid support to remove unbound antibody and probing said solid support with a reagent capable of selectively detecting human antibodies.

17. The method of claim 16, wherein said solid support is a plastic microtiter plate and said reagent capable of detecting human antibodies is anti-human IgG labelled with horseradish peroxidase which is reacted with a substrate which provides a color change end point in the presence of said peroxidase.

18. The method of claim 16, wherein said peptide as defined in claim 1 is HTLV-III env (487-511 )-Cys-NH₂.

19. The method of claim 16, wherein said peptide as defined in claim 1 is HTLV-III env Cys-(500-511 )-NH₂.

20. A process for the preparation of a vaccine comprising mixing a peptide as defined in claim 1 with a physiologically acceptable carrier.

21. A process according to claim 20, wherein said peptide is coupled to a toxin, which is utilized to confer immunity in humans.

22. A non-therapeutic process for the preparation of antibodies against AIDS virus comprising injecting a host with a sufficient amount of an immunogen as defined in claims 12 to 15 and recovering said antibodies from the serum of said host.

23. The use of a peptide as defined in claims 1 and 5 to 11 for the preparation of a protective immunization vaccine.

24. The use of a peptide as defined in claims 1 and 5 to 11 for testing human blood for the presence of antibodies to the AIDS virus.

25. The use of an immunogen composition as defined in claims 12 to 15 for the preparation of antibodies which selectively bind to a compound as defined in claims 1 and 5 to 11, HTLV-III env protein or AIDS virus.

26. A test kit for testing human blood or the presence of antibodies to the AIDS virus comprising in a container a peptide as defined in claims 1 and 5 to 11.

## Patentansprüche (Patentansprüche für folgende(n) Vertragsstaat(en) : BE, CH, DE, FR, GB, IT, LI, NL, SE)

1. Ein Peptid mit der Formel 
W-X-Lys-Ala-Lys-Arg-Arg-Val-Val-Gln-Arg-Glu-
Lys-Arg-Y-Z        (I)
 worin W H-, Cys-, oder Tyr- ist; X eine Bindung oder eine Subsequenz aus einer oder mehreren Aminosäuren ist, die am Carboxyl-Terminus von HTLV-III env (460-499) beginnt; Y eine Bindung oder eine Subsequenz aus einer oder mehreren Aminosäuren ist, die am Amino-Terminus von HTLV-lll env (512-550) beginnt; und Z -OH, -NH₂ oder -Cys-NH₂ ist, unter der Voraussetzung, dass eines von X oder Y eine Bindung ist und X verschieden von Pro-Thr ist.

2. Das Peptid gemäss Anspruch 1, worin sowohl X und Y Bindungen sind.

3. Das Peptid gemäss Anspruch 2, worin W H-und Z -Cys-NH₂ ist.

4. Das Peptid gemäss Anspruch 2, worin W Cys und Z -NH₂ ist.

5. Das Peptid gemäss Anspruch 2, worin W Tyrist.

6. Eine Verbindung gemäss Anspruch 5 markiert mit ¹²⁵l.

7. Das Peptid gemäss Anspruch 1, worin X eine Subsequenz aus einer oder mehreren Aminosäuren ist, die am Carboxyl-Terminus von HTLV-III env (460-499) beginnt, und Y eine Bindung ist.

8. Das Peptid gemäss Anspruch 7, worin X HTLV-III env (487-499) ist.

9. Das Peptid gemäss Anspruch 8, welches HTLV-!!! env (487-511) -Cys-NH₂ ist.

10. Das Peptid gemäss Anspruch 8, welches HTLV-lll env Tyr-(487-511)-Cys-NH₂ ist.

11. Eine immunogene Zusammensetzung enthaltend ein Peptid gemäss Anspruch 1, worin eines von W oder Z Cys ist, das kovalent an ein immunogenes Trägermaterial gebunden ist.

12. Die Zusammensetzung gemäss Anspruch 11, worin W H- und Z -Cys-NH₂ ist.

13. Die Zusammensetzung gemäss Anspruch 11, worin das besagte immunogene Trägermaterial Keyhole-Limpet-Hämocyanin ist.

14. Ein Peptid gemäss Anspruch 1 als Bestandteil eines Vakzins.

15. Ein Peptid gemäss Anspruch 1 als Bestandteil einer immunogenen Zusammensetzung.

16. Ein Verfahren für die Herstellung eines Peptides gemäss Anspruch 1, dadurch gekennzeichnet, dass konventionelle Peptidsynthesemethoden verwendet werden.

17. Ein Verfahren gemäss Anspruch 16, dadurch gekennzeichnet, dass Festphasen-Synthesemethoden verwendet werden.

18. Ein Verfahren für die Herstellung einer immunogenen Zusammensetzung gemäss Anspruch 11, dadurch gekennzeichnet, dass ein Peptid gemäss Anspruch 1, worin eines von W oder Z Cys ist, kovalent an ein immunogenes Trägermaterial gebunden ist.

19. Ein Verfahren gemäss Anspruch 18, dadurch gekennzeichnet, dass ein Peptid gemäss Anspruch 1, worin W H- und Z -Cys-NH₂ ist, verwendet wird.

20. Ein Verfahren gemäss Anspruch 18 oder 19, dadurch gekennzeichnet, dass als immunogenes Trägermaterial Schlüsselloch-Napfschnekken-Hemocyanin verwendet wird.

21. Ein Verfahren zum Testen von humanem Blut auf die Anwesenheit von Antikörpern für das virale ethiologische Agens von AIDS, umfassend Kontaktieren eines Peptides gemäss Anspruch 1, das an eine Festphase gebunden ist, mit einer Testprobe von humanem Blut, wobei jegliche Antikörper für das virale ethiologische Agens von AIDS, die in der besagten Testprobe vorhanden sind, an das besagte Peptid binden, Entfernen der besagten Testprobe, Waschen des besagten festen Trägers, um ungebundene Antikörper zu entfernen, und Untersuchen des besagten festen Trägers mit einem Reagenz, das in der Lage ist, selektiv humane Antikörper zu detektieren.

22. Das Verfahren gemäss Anspruch 21, worin der besagte feste Träger eine Plastik-Mikrotiterplatte ist und das zur Detektion von humanen Antikörpern fähige Reagenz mit Meerrettich-Peroxidase markiertes antihuman IgG ist, welches mit einem Substrat umgesetzt wird, das einen Farbwechselendpunkt in Gegenwart der besagten Peroxidase liefert.

23. Das Verfahren gemäss Anspruch 21, worin das besagte Peptid gemäss Anspruch 1 HTLV-III env (487-511)-Cys-NH₂ ist.

24. Das Verfahren gemäss Anspruch 21, worin das besagte Peptid gemäss Anspruch 1 HTLV-III env Cys-(500-511)-NH2 ist.

25. Ein Verfahren zur Herstellung eines Vakzins, umfassend Mischen eines Peptides gemäss Anspruch 1 mit einem physiologisch akzeptablen Träger.

26. Ein Verfahren gemäss Anspruch 25, worin das besagte Peptid an ein Toxin gekoppelt ist, welches dazu benutzt wird, Immunität in Menschen zu verleihen.

27. Ein nicht-therapeutisches Verfahren zur Herstellung von Antikörpern gegen AIDS-Viren, umfassend injizieren einer ausreichenden Menge eines Immunogens gemäss einem der Ansprüche 11 bis 13 in einen Wirt und Rückgewinnen der besagten Antikörper aus dem Serum des besagten Wirts.

28. Ein Vakzin gegen AIDS enthaltend ein Peptid gemäss Anspruch 1 in einem physiologisch akzeptablem Medium.

29. Das Vakzin gemäss Anspruch 28, worin das besagte Peptid an ein Toxin gekoppelt ist, das zur Verleihung von Immunität in Menschen verwendet wird.

30. Ein Antikörper erzeugt durch Immunisieren eines Wirts mit einem Immunogen gemäss Ansprüchen 11 bis 13, der selektiv an eine Verbindung gemäss Anspruch 1, HTLV-III env Protein oder AIDS-Virus bindet.

31. Der Antikörper gemäss Anspruch 30, welcher ein polyklonaler Antikörper ist.

32. Der Antikörper gemäss Anspruch 30, welcher ein monoclonaler Antikörper ist, der spezifisch für das Epitop HTLV-lll env (460-550) ist.

33. Die Verwendung eines Peptides gemäss Anspruch 1 zur Herstellung eines schützenden Immunisierungsvakzins.

34. Die Verwendung eines Peptides gemäss Anspruch 1 zum Testen von humanem Blut auf die Anwesenheit von Antikörpern für das AIDS-Virus.

35. Die Verwendung einer immunogenen Zusammensetzung gemäss Ansprüchen 11 bis 13 zur Herstellung von Antikörpern, die selektiv an eine Verbindung gemäss Anspruch 1, HTLV-III env Protein oder AIDS-Virus binden.

36. Ein wie in Anspruch 1 beanspruchtes Peptid, hergestellt durch ein wie in Anspruch 16 oder 17 beanspruchtes Verfahren.

37. Eine wie in einem der Ansprüche 11 bis 13 beanspruchte immunogene Zusammensetzung, hergestellt durch ein wie in einem der Ansprüche 18 bis 20 beanspruchtes Verfahren.

38. Ein Testkit zum Testen von humanen Blut auf die Anwesenheit von Antikörpern für das AIDS-Virus, enthaltend in einem Behälter ein Peptid gemäss Anspruch 1.

## Patentansprüche (Patentansprüche für folgende(n) Vertragsstaat(en) : AT)

1. Ein Verfahren zur Herstellung eines Peptides der Formel 
W-X-Lys-Ala-Lys-Arg-Arg-Val-Val-Gln-Arg-Glu-
Lys-Arg-Y-Z (I)
worin W H-, Cys-, oder Tyr- ist; X eine Bindung oder eine Subsequenz aus einer oder mehreren Aminosäuren ist, die am Carboxyl-Terminus von HTLV-III env (460-499) beginnt; Y eine Bindung oder eine Subsequenz aus einer oder mehreren Aminosäuren ist, die am Amino-Terminus von HTLV-lll env (512-550) beginnt; und Z -OH, -NH₂ oder -Cys-NH₂ ist, unter der Voraussetzung, dass eines von X oder Y eine Bindung ist und X verschieden von Pro-Thr ist, durch übliche Peptid-Synthesemethoden.

2. Ein Verfahren gemäss Anspruch 1, dadurch charakterisiert, dass Festphasen-Synthesemethoden verwendet werden.

3. Ein Verfahren gemäss Anspruch 2, dadurch charakterisiert, dass Schutzgruppen und/oder die Festphasen-Harze, die während der Festphasensynthese eines Peptides gemäss Anspruch 1 eingeführt wurden, durch übliche Mittel abgespalten werden.

4. Ein Verfahren gemäss Anspruch 3, dadurch charakterisiert, dass die Spaltung durch Behandlung mit wasserfreier, flüssiger Flusssäure durchgeführt wird.

5. Ein Verfahren gemäss einem der Ansprüche 1 bis 4, dadurch gekennzeichnet, dass ein wie in Anspruch 1 definiertes Peptid, worin X und Y beides Bindungen sind, W H- und Z -Cys-NH₂ ist, hergestellt wird.

6. Ein Verfahren gemäss einem der Ansprüche 1 bis 4, dadurch gekennzeichnet, dass ein wie in Anspruch 1 definiertes Peptid, worin X und Y beides Bindungen sind, W Cys und Z -NH₂ ist, hergestellt wird.

7. Ein Verfahren gemäss einem der Ansprüche 1 bis 4, dadurch gekennzeichnet, dass ein wie in Anspruch 1 definiertes Peptid, worin X und Y beides Bindungen sind und W Tyr- ist, hergestellt wird.

8. Ein Verfahren gemäss einem der Ansprüche 1 bis 4, dadurch gekennzeichnet, dass ein wie in Anspruch 1 definiertes Peptid, worin X eine Subsequenz aus einer oder mehreren Aminosäuren ist, die am Carboxyl-Terminus von HTLV-III env (460-499) beginnt, und Y eine Bindung ist, hergestellt wird.

9. Ein Verfahren gemäss einem der Ansprüche 1 bis 4, dadurch gekennzeichnet, dass ein wie in Anspruch 1 definiertes Peptid, worin X HTLV-III env (487-499) ist und Y eine Bindung ist, hergestellt wird.

10. Ein Verfahren gemäss einem der Ansprüche 1 bis 4, dadurch gekennzeichnet, dass ein HTLV-III env (487-511)-Cys-NH₂ Peptid hergestellt wird.

11. Ein Verfahren gemäss einem der Ansprüche 1 bis 4, dadurch gekennzeichnet, dass ein HTLV-!!! env (Tyr-(487-511)-Cys-NH₂ Peptid hergestellt wird.

12. Ein Verfahren für die Herstellung einer Immunogen-Zusammensetzung, dadurch gekennzeichnet, dass ein wie in Anspruch 1 definiertes Peptid, worin W oder Z Cys ist, kovalent an ein immunogenes Trägermaterial gebunden ist.

13. Ein Verfahren gemäss Anspruch 12, dadurch gekennzeichnet, dass ein wie in Anspruch 1 definiertes Peptid, das W als H- und Z als -Cys-NH₂ hat, verwendet wird.

14. Ein Verfahren gemäss Anspruch 12 oder 13, dadurch gekennzeichnet, dass die besagten Peptide an das besagte immunogene Trägermaterial über eine übliche bifunktionale Linkergruppe gebunden sind.

15. Ein Verfahren gemäss Ansprüchen 12 bis 14, dadurch gekennzeichnet, dass als immunogenes Trägermaterial Keyhole-Limpet-Hämocyanin verwendet wird.

16. Ein Verfahren zum Testen von humanem Blut auf die Anwesenheit von Antikörpern für das virale ethiologische Agens von AIDS, umfassend Kontaktieren eines Peptides gemäss Anspruch 1, das an eine Festphase gebunden ist, mit einer Testprobe von humanem Blut, wobei jegliche Antikörper für das virale ethiologische Agens von AIDS, die in der besagten Testprobe vorhanden sind, an das besagte Peptid binden, Entfernen der besagten Testprobe, Waschen des besagten festen Trägers, um ungebundene Antikörper zu entfernen, und Untersuchen des besagten festen Trägers mit einem Reagenz, das in der Lage ist, selektiv humane Antikörper zu detektieren.

17. Das Verfahren gemäss Anspruch 16, worin der besagte feste Träger eine Plastik-Mikrotiterplatte ist und das zur Detektion von humanen Antikörpern fähige Reagenz mit Meerrettich-Peroxidase markiertes antihuman IgG ist, welches mit einem Substrat umgesetzt wird, das einen Farbwechselendpunkt in Gegenwart der besagten Peroxidase liefert.

18. Das Verfahren gemäss Anspruch 16, worin das besagte Peptid gemäss Anspruch 1 HTLV-III env (487-511)-Cys-NH₂ ist.

19. Das Verfahren gemäss Anspruch 16, worin das besagte Peptid gemäss Anspruch 1 HTLV-III env Cys-(500-511)-NH2 ist.

20. Ein Verfahren zur Herstellung eines Vakzins umfassend Mischen eines Peptides gemäss Anspruch 1 mit einem physiologisch akzeptablen Träger.

21. Ein Verfahren gemäss Anspruch 20, worin das besagte Peptid an ein Toxin gekoppelt ist, welches dazu benutzt wird, Immunität in Menschen zu verleihen.

22. Ein nicht-therapeutisches Verfahren zur Herstellung von Antikörpern gegen AIDS-Viren, umfassend injizieren einer ausreichenden Menge eines Immunogens gemäss einem der Ansprüche 11 bis 13 in einem Wirt und Rückgewinnen der besagten Antikörper aus dem Serum des besagten Wirts.

23. Die Verwendung eines Peptides wie in Ansprüchen 1 und 5 bis 11 definiert, zur Herstellung eines schützenden immunisierenden Vakzins.

24. Die Verwendung eines Peptides wie in Ansprüchen 1 und 5 bis 11 definiert, zum Testen von humanem Blut auf die Gegenwart von Antikörpern für das AIDS-Virus.

25. Die Verwendung einer wie in Ansprüchen 12 bis 15 definierten Immunogen-Zusammensetzung, zur Herstellung von Antikörpern, die selektiv an eine wie in Ansprüchen 1 und 5 bis 11 definierte Verbindung, HTLV-III env Protein oder AIDS-Virus binden.

26. Ein Testkit zum Testen von humanem Blut auf die Anwesenheit von Antikörpern für das AIDS-Virus, enthaltend in einem Behälter ein Peptid wie in Ansprüchen 1 und 5 bis 11 definiert.

## Patentansprüche (Patentansprüche für folgende(n) Vertragsstaat(en) : ES)

1. Ein Verfahren zur Herstellung eines Peptides der Formel 
W-X-Lys-Ala-Lys-Arg-Arg-Val-Val-Gln-Arg-Glu-
Lys-Arg-Y-Z        (I)
 worin W H-, Cys-, oder Tyr- ist; X eine Bindung oder eine Subsequenz aus einer oder mehreren Aminosäuren ist, die am Carboxyl-Terminus von HTLV-III env (460-499) beginnt; Y eine Bindung oder eine Subsequenz aus einer oder mehreren Aminosäuren ist, die am Amino-Terminus von HTLV-lll env (512-550) beginnt; und Z -OH, -NH₂ oder -Cys-NH₂ ist, unter der Voraussetzung, dass eines von X oder Y eine Bindung ist, durch übliche Peptid-Synthesemethoden.

2. Ein Verfahren gemäss Anspruch 1, dadurch charakterisiert, dass Festphasen-Synthesemethoden verwendet werden.

3. Ein Verfahren gemäss Anspruch 2, dadurch charakterisiert, dass Schutzgruppen und/oder die Festphasen-Harze, die während der Festphasensynthese eines Peptides gemäss Anspruch 1 eingeführt wurden, durch übliche Mittel abgespalten werden.

4. Ein Verfahren gemäss Anspruch 3, dadurch charakterisiert, dass die Spaltung durch Behandlung mit wasserfreier, flüssiger Flusssäure durchgeführt wird.

5. Ein Verfahren gemäss einem der Ansprüche 1 bis 4, dadurch gekennzeichnet, dass ein wie in Anspruch 1 definiertes Peptid, worin X und Y beides Bindungen sind, W H- und Z -Cys-NH₂ ist, hergestellt wird.

6. Ein Verfahren gemäss einem der Ansprüche 1 bis 4, dadurch gekennzeichnet, dass ein wie in Anspruch 1 definiertes Peptid, worin X und Y beides Bindungen sind, W Cys und Z -NH₂ ist, hergestellt wird.

7. Ein Verfahren gemäss einem der Ansprüche 1 bis 4, dadurch gekennzeichnet, dass ein wie in Anspruch 1 definiertes Peptid, worin X und Y beides Bindungen sind und W Tyr- ist, hergestellt wird.

8. Ein Verfahren gemäss einem der Ansprüche 1 bis 4, dadurch gekennzeichnet, dass ein wie in Anspruch 1 definiertes Peptid, worin X eine Subsequenz aus einer oder mehreren Aminosäuren ist, die am Carboxyl-Terminus von HTLV-III env (460-499) beginnt, und Y eine Bindung ist, hergestellt wird.

9. Ein Verfahren gemäss einem der Ansprüche 1 bis 4, dadurch gekennzeichnet, dass ein wie in Anspruch 1 definiertes Peptid, worin X HTLV-III env (487-499) ist und Y eine Bindung ist, hergestellt wird.

10. Ein Verfahren gemäss einem der Ansprüche 1 bis 4, dadurch gekennzeichnet, dass ein HTLV-III env (487-511)-Cys-NH₂ Peptid hergestellt wird.

11. Ein Verfahren gemäss einem der Ansprüche 1 bis 4, dadurch gekennzeichnet, dass ein HTLV-III env (Tyr-(487-511)-Cys-NH₂ Peptid hergestellt wird.

12. Ein Verfahren für die Herstellung einer Immunogen-Zusammensetzung, dadurch gekennzeichnet, dass ein wie in Anspruch 1 definiertes Peptid, worin W oder Z Cys ist, kovalent an ein immunogenes Trägermaterial gebunden ist.

13. Ein Verfahren gemäss Anspruch 12, dadurch gekennzeichnet, dass ein wie in Anspruch 1 definiertes Peptid, das W als H- und Z als -Cys-NH₂ hat, verwendet wird.

14. Ein Verfahren gemäss Anspruch 12 oder 13, dadurch gekennzeichnet, dass die besagten Peptide an das besagte immunogene Trägermaterial über eine übliche bifunktionale Linkergruppe gebunden sind.

15. Ein Verfahren gemäss Ansprüchen 12 bis 14, dadurch gekennzeichnet, dass als immunogenes Trägermaterial Keyhole-Limpet-Hämocyanin verwendet wird.

16. Ein Verfahren zum Testen von humanem Blut auf die Anwesenheit von Antikörpern für das virale ethiologische Agens von AIDS, umfassend Kontaktieren eines Peptides gemäss Anspruch 1, das an eine Festphase gebunden ist, mit einer Testprobe von humanem Blut, wobei jegliche Antikörper für das virale ethiologische Agens von AIDS, die in der besagten Testprobe vorhanden sind, an das besagte Peptid binden, Entfernen der besagten Testprobe, Waschen des besagten festen Trägers, um ungebundene Antikörper zu entfernen, und Untersuchen des besagten festen Trägers mit einem Reagenz, das in der Lage ist, selektiv humane Antikörper zu detektieren.

17. Das Verfahren gemäss Anspruch 16, worin der besagte feste Träger eine Plastik-Mikrotiterplatte ist und das zur Detektion von humanen Antikörpern fähige Reagenz mit Meerrettich-Peroxidase markiertes antihuman IgG ist, welches mit einem Substrat umgesetzt wird, das einen Farbwechselendpunkt in Gegenwart der besagten Peroxidase liefert.

18. Das Verfahren gemäss Anspruch 16, worin das besagte Peptid gemäss Anspruch 1 HTLV-III env (487-511)-Cys-NH₂ ist.

19. Das Verfahren gemäss Anspruch 16, worin das besagte Peptid gemäss Anspruch 1 HTLV-III env Cys-(500-511)-NH2 ist.

20. Ein Verfahren zur Herstellung eines Vakzins umfassend Mischen eines Peptides gemäss Anspruch 1 mit einem physiologisch akzeptablen Träger.

21. Ein Verfahren gemäss Anspruch 20, worin das besagte Peptid an ein Toxin gekoppelt ist, welches dazu benutzt wird, Immunität in Menschen zu verleihen.

22. Ein nicht-therapeutisches Verfahren zur Herstellung von Antikörpern gegen AIDS-Viren, umfassend injizieren einer ausreichenden Menge eines Immunogens gemäss einem der Ansprüche 11 bis 13 in einem Wirt und Rückgewinnen der besagten Antikörper aus dem Serum des besagten Wirts.

23. Die Verwendung eines Peptides wie in Ansprüchen 1 und 5 bis 11 definiert, zur Herstellung eines schützenden immunisierenden Vakzins.

24. Die Verwendung eines Peptides wie in Ansprüchen 1 und 5 bis 11 definiert, zum Testen von humanem Blut auf die Gegenwart von Antikörpern für das AIDS-Virus.

25. Die Verwendung einer wie in Ansprüchen 12 bis 15 definierten Immunogen-Zusammensetzung, zur Herstellung von Antikörpern, die selektiv an eine wie in Ansprüchen 1 und 5 bis 11 definierte Verbindung, HTLV-III env Protein oder AIDS-Virus binden.

26. Ein Testkit zum Testen von humanem Blut auf die Anwesenheit von Antikörpern für das AIDS-Virus, enthaltend in einem Behälter ein Peptid wie in Ansprüchen 1 und 5 bis 11 definiert.

## Revendications (Revendications pour l'(les) Etat(s) contractant(s) suivant(s) : suivants : BE, CH, DE, FR, GB, IT, LI, NL, SE)

1. Peptide de formule 
W-X-Lys-Ala-Lys-Arg-Arg-Val-Val-Gln-Arg-Glu-
Lys-Arg-Y-Z (I)
 dans laquelle W est H-, Cys- ou Tyr-; X est une liaison ou une sous-séquence d'un ou plusieurs aminoacides partant de l'extrémité carboxy-terminale de HTLV-III env (460-499); Y est une liaison ou une sous-séquence d'un ou plusieurs aminoacides partant de l'extrémité amino-terminale de HTLV-lll env (512-550); et Z est -OH, -NH₂ ou -Cys-NH₂, étant entendu, cependant, que l'un des restes X et Y est une liaison et que X est différent de Pro-Thr.

2. Peptide de la revendication 1, dans lequel X et Y sont des liaisons.

3. Peptide de la revendication 2, dans lequel W est H-et Z est -Cys-NH₂.

4. Peptide de la revendication 2, dans lequel W est Cys et Z est-NH2.

5. Peptide de la revendication 2, dans lequel W est Tyr-.

6. Composé de la revendication 5, marqué au ₁2⁵1.

7. Peptide de la revendication 1, dans lequel X est une sous-séquence d'un ou plusieurs aminoacides partant de l'extrémité carboxy-terminale de HTLV-III env (460-499) et Y est une liaison.

8. Peptide de la revendication 7, dans lequel X est HTLV-III env (487-499).

9. Peptide de la revendication 8, qui est HTLV-III env (487-511)-Cys-NH₂.

10. Peptide de la revendication 8, qui est HTLV-III env Tyr-(487-511)-Cys-NH₂.

11. Composition immunogène comprenant un peptide selon la revendication 1, dans laquelle l'un des restes W et Z est un reste Cys lié par covalence à une substance porteuse immunogène.

12. Composition de la revendication 11, dans laquelle W est H- et Z est -Cys-NH₂.

13. Composition de la revendication 11, dans laquelle ladite substance porteuse immunogène est l'hémocyanine de patelle.

14. Peptide de la revendication 1, en tant que composant d'un vaccin.

15. Peptide de la revendication 1, an tant que composant d'une composition immunogène.

16. Procédé pour la préparation d'un peptide selon la revendication 1, caractérisé en ce que l'on utilise des méthodes classiques de synthèse de peptides.

17. Procédé selon la revendication 16, caractérisé en ce que l'on utilise des méthodes de synthèse en phase solide.

18. Procédé pour la préparation d'une composition immunogène de la revendication 11, caractérisé en ce qu'un peptide de la revendication 1, dans lequel l'un des restes W ou Z est Cys, est lié par covalence à une substance porteuse immunogène.

19. Procédé selon la revendication 18, caractérisé en ce que l'on utilise un peptide de la revendication 1 dans lequel W est H- et Z est -Cys-NH₂.

20. Procédé selon la revendication 18 ou 19, caractérisé en ce que, comme substance porteuse immunogène, on utilise de l'hémocyanine de patelle.

21. Procédé d'essai de sang humain pour la détection de la présence d'anticorps dirigés contre l'agent viral étiologique du SIDA, qui comprend la mise en contact d'un peptide de la revendication 1, qui est fixé sur un support solide, avec un échantillon de sang humain d'essai, ce par quoi n'importe quels anticorps dirigés contre l'agent viral étiologique du SIDA, présents dans ledit échantillon d'essai, se lient audit peptide, l'élimination dudit échantillon d'essai, le lavage dudit support solide pour l'élimination des anticorps non liés, et l'essai dudit support solide au moyen d'un réactif capable de détecter sélectivement des anticorps humains.

22. Procédé de la revendication 21, dans lequel ledit support solide est une plaque de microti- trage en matière plastique et ledit réactif capable de détecter des anticorps humains est un anticorps anti-IgG humaine marqué à la peroxydase de raifort, qui est mis en réaction avec un substrat qui donne un virage coloré en présence de ladite peroxydase.

23. Procédé de la revendication 21, dans lequel ledit peptide de la revendication 1 est HTLV-III env (487-511)-Cys-NH₂.

24. Procédé de la revendication 21, dans lequel ledit peptide de la revendication 1 est HTLV-III env Cys-(500-511)-NH2.

25. Procédé pour la préparation d'un vaccin, comprenant le mélange d'un peptide de la revendication 1 avec un véhicule physiologiquement acceptable.

26. Procédé selon la revendication 25, dans lequel ledit peptide est couplé avec une toxine qui est utilisée pour conférer une immunité à l'homme.

27. Procédé non thérapeutique pour la production d'anticorps dirigés contre le virus du SIDA, comprenant l'injection à un hôte d'une quantité suffisante d'un immunogène des revendications 11 à 13, et la récupération desdits anticorps à partir du sérum dudit hôte.

28. Vaccin contre le SIDA, comprenant un peptide de la revendication 1, dans un milieu physiologiquement acceptable.

29. Vaccin de la revendication 28, dans lequel ledit peptide est couplé avec une toxine utilisée pour conférer une immunité chez l'homme.

30. Vaccin suscité par immunisation d'un hôte avec un immunogène des revendications 11 à 13, qui se lie sélectivement à un composé de la revendication 1, la protéine HTLV-III env ou le virus du SIDA.

31. Anticorps de la revendication 30, qui est un anticorps polyclonal.

32. Anticorps de la revendication 30, qui est un anticorps monoclonal spécifique de l'épitope HTLV-III env (460-550).

33. Utilisation d'un peptide de la revendication 1, pour la préparation d'un vaccin d'immunisation protectrice.

34. Utilisation d'un peptide de la revendication 1, pour tester du sang humain pour détecter la présence d'anticorps dirigés contre le virus du SIDA.

35. Utilisation d'une composition immunogène des revendications 11 à 13, pour la production d'anticorps qui se lient sélectivement à un composé de la revendication 1, la protéine HTLV-III env ou le virus du SIDA.

36. Peptide selon la revendication 1, préparé par un procédé tel que revendiqué dans la revendication 16 ou 17.

37. Composition immunogène selon l'une quelconque des revendications 11 à 13, préparée par un procédé tel que revendiqué dans l'une quelconque des revendications 18 à 20.

38. Nécessaire d'essai pour tester du sang humain pour détecter la présence d'anticorps dirigés contre le virus du SIDA, comprenant, dans un contenant, un peptide de la revendication 1.

## Revendications (Revendications pour l'(les) Etat(s) contractant(s) suivant(s) : suivant : AT)

1. Procédé pour la préparation d'un peptide de formule 
W-X-Lys-Ala-Lys-Arg-Arg-Val-Val-Gln-Arg-Glu-
Lys-Arg-Y-Z (I)
 dans laquelle W est H-, Cys- ou Tyr-; X est une liaison ou une sous-séquence d'un ou plusieurs aminoacides partant de l'extrémité carboxy-terminale de HTLV-III env (460-499); Y est une liaison ou une sous-séquence d'un ou plusieurs aminoacides partant de l'extrémité amino-terminale de HTLV-!!! env (512-550); et Z est -OH, -NH₂ ou -Cys-NH₂, étant entendu, cependant, que l'un des restes X et Y est une liaisonet que X est différent de Pro-Thr, par des méthodes classiques de synthèse des peptides.

2. Procédé selon la revendication 1, caractérisé en ce que l'on utilise des méthodes de synthèse en phase solide.

3. Procédé selon la revendication 2, caractérisé en ce que des groupes protecteurs et/ou les résines en phase solide introduits au cours de la synthèse en phase solide d'un peptide de la revendication 1 sont séparés par des moyens classiques.

4. Procédé selon la revendication 3, caractérisé en ce que la séparation est effectuée partraite- ment par de l'acide fluorhydrique liquide anhydre.

5. Procédé selon l'une quelconque des revendications 1 à 4, caractérisé en ce que l'on prépare un peptide tel que défini dans la revendication 1, dans lequel X et Y sont l'un et l'autre des liaisons, W est H- et Z est -Cys-NH₂.

6. Procédé selon l'une quelconque des revendications 1 à 4, caractérisé en ce que l'on prépare un peptide tel que défini dans la revendication 1, dans lequel X et Y sont l'un et l'autre des liaisons, W est Cys et Z est -NH₂.

7. Procédé selon l'une quelconque des revendications 1 à 4, caractérisé en ce que l'on prépare un peptide tel que défini dans la revendication 1, dans lequel X et Y sont l'un et l'autre des liaisons et W est Tyr-.

8. Procédé selon l'une quelconque des revendications 1 à 4, caractérisé en ce que l'on prépare un peptide tel que défini dans la revendication 1, dans lequel X est une sous-séquence d'un ou plusieurs aminoacides partant de l'extrémité carboxy-terminale de HTLV-!!! env (460-499) et Y est une liaison.

9. Procédé selon l'une quelconque des revendications 1 à 4, caractérisé en ce que l'on prépare un peptide tel que défini dans la revendication 1, dans lequel X est HTLV-III env (487-499) et Y est une liaison.

10. Procédé selon l'une quelconque des revendications 1 à 4, caractérisé en ce que l'on prépare un peptide HTLV-lll env (487-511)-Cys-NH₂.

11. Procédé selon l'une quelconque des revendications 1 à 4, caractérisé en ce que l'on prépare un peptide HTLV-lll env Tyr-(487-511)-Cys-NH₂.

12. Procédé pour la préparation d'une composition immunogène, caractérisé en ce qu'un peptide tel que défini dans la revendication 1, dans lequel W ou Z est un reste Cys, est lié par covalence à une substance porteuse immunogène.

13. Procédé selon la revendication 12, caractérisé en ce que l'on utilise un peptide tel que défini dans la revendication 1, dans lequel W est H-et Z est -Cys-NH₂.

14. Procédé selon la revendication 12 ou 13, caractérisé en ce que lesdits peptides sont liés à ladite substance porteuse immunogène par l'intermédiaire d'un groupe de liaison bifonc- tionnel.

15. Procédé selon les revendications 12 à 14, caractérisé en ce que, comme substance porteuse immunogène, on utilise de l'hémocyanine de patelle.

16. Procédé d'essai de sang humain pour la détection de la présence d'anticorps dirigés contre l'agent viral étiologique du SIDA, qui comprend la mise en contact d'un peptide tel que défini dans la revendication 1, qui est fixé sur un support solide, avec un échantillon de sang humain d'essai, ce par quoi n'importe quels anticorps dirigés contre l'agent viral étiologique du SIDA, présents dans ledit échantillon d'essai, se lient audit peptide, l'élimination dudit échantillon d'essai, le lavage dudit support solide pour l'élimination des anticorps non liés, et l'essai dudit support solide au moyen d'un réactif capable de détecter sélectivement des anticorps humains.

17. Procédé de la revendication 16, dans lequel ledit support solide est une plaque de microti- trage en matière plastique et ledit réactif capable de détecter des anticorps humains est un anticorps anti-IgG humaine marqué à la peroxydase de raifort, qui est mis en réaction avec un substrat qui donne un virage coloré en présence de ladite peroxydase.

18. Procédé de la revendication 16, dans lequel ledit peptide tel que défini dans la revendication 1 est HTLV-!!! env (487-511)-Cys-NH₂.

19. Procédé de la revendication 21, dans lequel ledit peptide tel que défini dans la revendication 1 est HTLV-lll env Cys-(500-511)-NH₂.

20. Procédé pour la préparation d'un vaccin, comprenant le mélange d'un peptide tel que défini dans la revendition 1, avec un véhicule physiologiquement acceptable.

21. Procédé selon la revendication 20, dans lequel ledit peptide est couplé avec une toxine qui est utilisée pour conférer une immunité à l'homme.

22. Procédé non thérapeutique pour la production d'anticorps dirigés contre le virus du SIDA, comprenant l'injection à un hôte d'une quantité suffisante d'un immunogène tel que défini dans les revendications 12 à 15, et la récupération desdits anticorps à partir du sérum dudit hôte.

23. Utilisation d'un peptide tel que défini dans les revendications 1 et 5 à 11, pour la préparation d'un vaccin d'immunisation protectrice.

24. Utilisation d'un peptide tel que défini dans les revendications 1 et 5 à 11, pour tester du sang humain pour détecter la présence d'anticorps dirigés contre le virus du SIDA.

25. Utilisation d'une composition immunogène telle que définie dans les revendications 12 à 15, pour la production d'anticorps qui se lient sélectivement à un composé tel que défini dans les revendications 1 et 5 à 11, la protéine HTLV-III env ou le virus du SIDA.

26. Nécessaire d'essai pour tester du sang humain pour détecter la présence d'anticorps dirigés contre le virus du SIDA, comprenant, dans un contenant, un peptide tel que défini dans les revendications 1 et 5 à 11.

## Revendications (Revendications pour l'(les) Etat(s) contractant(s) suivant(s) : suivant : ES)

1. Procédé pour la préparation d'un peptide de formule 
W-X-Lys-Ala-Lys-Arg-Arg-Val-Val-Gln-Arg-Glu-
Lys-Arg-Y-Z (I)
 dans laquelle W est H-, Cys- ou Tyr-; X est une liaison ou une sous-séquence d'un ou plusieurs aminoacides partant de l'extrémité carboxy-terminale de HTLV-III env (460-499); Y est une liaison ou une sous-séquence d'un ou plusieurs aminoacides partant de l'extrémité amino-terminale de HTLV-lll env (512-550); et Z est -OH, -NH₂ ou -Cys-NH₂, étant entendu, cependant, que l'un des restes X et Y est une liaison, par des méthodes classiques de synthèse des peptides.

2. Procédé selon la revendication 1, caractérisé en ce que l'on utilise des méthodes de synthèse en phase solide.

3. Procédé selon la revendication 2, caractérisé en ce que des groupes protecteurs et/ou les résines en phase solide introduits au cours de la synthèse en phase solide d'un peptide de la revendication 1 sont séparés par des moyens classiques.

4. Procédé selon la revendication 3, caractérisé en ce que la séparation est effectuée par traitement par de l'acide fluorhydrique liquide anhydre.

5. Procédé selon l'une quelconque des revendications 1 à 4, caractérisé en ce que l'on prépare un peptide tel que défini dans la revendication 1, dans lequel X et Y sont l'un et l'autre des liaisons, W est H- et Z est -Cys-NH₂.

6. Procédé selon l'une quelconque des revendications 1 à 4, caractérisé en ce que l'on prépare un peptide tel que défini dans la revendication 1, dans lequel X et Y sont l'un et l'autre des liaisons, W est Cys et Z est -NH₂.

7. Procédé selon l'une quelconque des revendications 1 à 4, caractérisé en ce que l'on prépare un peptide tel que défini dans la revendication 1, dans lequel X et Y sont l'un et l'autre des liaisons et W est Tyr-.

8. Procédé selon l'une quelconque des revendications 1 à 4, caractérisé en ce que l'on prépare un peptide tel que défini dans la revendication 1, dans lequel X est une sous-séquence d'un ou plusieurs aminoacides partant de l'extrémité carboxy-terminale de HTLV-!!! env (460-499) et Y est une liaison.

9. Procédé selon l'une quelconque des revendications 1 à 4, caractérisé en ce que l'on prépare un peptide tel que défini dans la revendication 1, dans lequel X est HTLV-III env (487-499) et Y est une liaison.

10. Procédé selon l'une quelconque des revendications 1 à 4, caractérisé en ce que l'on prépare un peptide HTLV-lll env (487-511)-Cys-NH₂.

11. Procédé selon l'une quelconque des revendications 1 à 4, caractérisé en ce que l'on prépare un peptide HTLV-lll env Tyr-(487-511)-Cys-NH₂.

12. Procédé pour la préparation d'une composition immunogène, caractérisé en ce qu'un peptide tel que défini dans la revendication 1, dans lequel W ou Z est un reste Cys, est lié par covalence à une substance porteuse immunogène.

13. Procédé selon la revendication 12, caractérisé en ce que l'on utilise un peptide tel que défini dans la revendication 1, dans lequel W est H-et Z est -Cys-NH₂.

14. Procédé selon la revendication 12 ou 13, caractérisé en ce que lesdits peptides sont liés à ladite substance porteuse immunogène par l'intermédiaire d'un groupe de liaison bifonc- tionnel.

15. Procédé selon les revendications 12 à 14, caractérisé en ce que, comme substance porteuse immunogène, on utilise de l'hémocyanine de patelle.

16. Procédé d'essai de sang humain pour la détection de la présence d'anticorps dirigés contre l'agent viral étiologique du SIDA, qui comprend la mise en contact d'un peptide tel que défini dans la revendication 1, qui est fixé sur un support solide, avec un échantillon de sang humain d'essai, ce par quoi n'importe quels anticorps dirigés contre l'agent viral étiologique du SIDA, présents dans ledit échantillon d'essai, se lient audit peptide, l'élimination dudit échantillon d'essai, le lavage dudit support solide pour l'élimination des anticorps non liés, et l'essai dudit support solide au moyen d'un réactif capable de détecter sélectivement des anticorps humains.

17. Procédé de la revendication 16, dans lequel ledit support solide est une plaque de microti- trage en matière plastique et ledit réactif capable de détecter des anticorps humains est un anticorps anti-IgG humaine marqué à la peroxydase de raifort, qui est mis en réaction avec un substrat qui donne un virage coloré en présence de ladite peroxydase.

18. Procédé de la revendication 16, dans lequel ledit peptide tel que défini dans la revendication 1 est HTLV-lll env (487-511)-Cys-NH₂.

19. Procédé de la revendication 21, dans lequel ledit peptide tel que défini dans la revendication 1 est HTLV-lll env Cys-(500-511)-NH₂.

20. Procédé pour la préparation d'un vaccin, comprenant le mélange d'un peptide tel que défini dans la revendition 1, avec un véhicule physiologiquement acceptable.

21. Procédé selon la revendication 20, dans lequel ledit peptide est couplé avec une toxine qui est utilisée pour conférer une immunité à l'homme.

22. Procédé non thérapeutique pour la production d'anticorps dirigés contre le virus du SIDA, comprenant l'injection à un hôte d'une quantité suffisante d'un immunogène tel que défini dans les revendications 12 à 15, et la récupération desdits anticorps à partir du sérum dudit hôte.

23. Utilisation d'un peptide tel que défini dans les revendications 1 et 5 à 11, pour la préparation d'un vaccin d'immunisation protectrice.

24. Utilisation d'un peptide tel que défini dans les revendications 1 et 5 à 11, pour tester du sang humain pour détecter la présence d'anticorps dirigés contre le virus du SIDA.

25. Utilisation d'une composition immunogène telle que définie dans les revendications 12 à 15, pour la production d'anticorps qui se lient sélectivement à un composé tel que défini dans les revendications 1 et 5 à 11, la protéine HTLV-III env ou le virus du SIDA.

26. Nécessaire d'essai pour tester du sang humain pour détecter la présence d'anticorps dirigés contre le virus du SIDA, comprenant, dans un contenant, un peptide tel que défini dans les revendications 1 et 5 à 11.
